(19) Europäisches Patentamt
European Patent Office
Office européen des brevets

(11) Numéro de publication : **0 069 622 B1**

# (12) FASCICULE DE BREVET EUROPÉEN

(45) Date de publication du fascicule du brevet : **18.09.85**

(21) Numéro de dépôt : **82401118.3**

(22) Date de dépôt : **18.06.82**

(51) Int. Cl.⁴ : **C 07 C 85/24**, C 07 D333/28, C 07 C 87/28, C 07 D213/18// C07F9/40, C07F9/24, C07F9/32, C07F9/44

## (54) Procédé de préparation d'éthylamines Beta-cyclo-substituées et produits ainsi obtenus.

(30) Priorité : **30.06.81 FR 8113065**

(43) Date de publication de la demande : **12.01.83 Bulletin 83/02**

(45) Mention de la délivrance du brevet : **18.09.85 Bulletin 85/38**

(84) Etats contractants désignés : **AT BE CH DE FR GB IT LI LU NL SE**

(56) Documents cités :
**US-A- 2 983 729**
**TETRAHEDRON LETTERS, no.26, 1979, Pergamon Press Ltd., (GB) N.L.J.M. BROEKHOF et al.: "Enamine synthesis by the horner-wittig reaction", pages 2433-2436.**
**JUSTUS LIEBIGS ANNALEN DER CHEMIE, vol.686, 1965, Weinheim (DE) H. ZIMMER et al.: "Synthesen mit Alpha-Amino-Alkyl-Phosphonsäureestern", pages 107-114.**
**CHEMICAL ABSTRACTS, vol.73, no.19, 9 novembre 1970, page 366, résumé no.98839k, Columbus, Ohio (US) S. GRONOWITZ et al.: "Thiophene isosteres of isoquinoline. I.Synthesis of thieno (2,3-c) pyridines and thieno(3,2-c)pyridines"**

(73) Titulaire : **SANOFI, société anonyme
40, Avenue George V
F-75008 Paris (FR)**

(72) Inventeur : **Chekroun, Isaac
26 Rue Peyras
F-31000 Toulouse (FR)**
Inventeur : **Heymes, Alain
Chemin de l'Adrech
F-04200 Sisteron (FR)**

(74) Mandataire : **Polus, Camille et al
c/o Cabinet Lavoix 2, Place d'Estienne d'Orves
F-75441 Paris Cedex 09 (FR)**

**EP 0 069 622 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention est relative à un nouveau procédé de préparation d'éthylamines β-cyclo-substituées, répondant à la formule I :

$$Ar—CH_2—CH_2—NH_2 \qquad (I)$$

dans laquelle :

Ar représente un radical aromatique hétérocyclique ou non tel que thiényle, furyle, pyridyle, phényle, naphtyle, éventuellement mono- ou polysubstitué par des groupes tels que halogène, nitro, amino, cyano, carboxy, alcoyle, halogéno-alcoyle, alcoxy, halogéno-alcoxy ou phényle.

Un grand nombre de dérivés répondant à la formule I sont connus et utilisés comme intermédiaires dans la préparation de composés employés aussi bien dans l'industrie chimique que pharmaceutique.

C'est ainsi, qu'à titre d'exemple, on peut mentionner parmi les dérivés obtenus selon le nouveau procédé de préparation les (thiényl-2)- et (thiényl-3)-2 éthylamines qui, par des moyens connus et faciles à mettre en œuvre (cf. S. Gronowitz et E. Sandberg, Arkiv. Kemi, *32,* 217 (1970)) conduisent respectivement aux tétrahydro-4,5,6,7 thiéno [3,2-c]- et [2,3-c] pyridines dont certains dérivés ont fait l'objet de la part de la présente demanderesse pour leurs applications en thérapeutique et/ou leurs procédés de préparation de plusieurs brevets (Fr.73.03503, Fr.75.03968, Fr.75.20241, Fr.75.23786, Fr.75.24486, Fr.76.00003, Fr.77.21517).

L'invention a pour objet un procédé simple et peu coûteux, en regard de la technique antérieure d'obtention des composés de formule I.

Conformément au procédé de l'invention, pour préparer les dérivés de formule I :

a) on condense un dérivé de formule II :

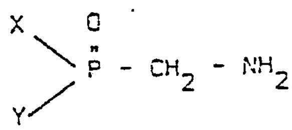

(II)

dans laquelle X et Y représentent ensemble ou séparément un radical alkyle, aryle, alkoxy, ariloxy, dialkyl ou diarylamino de telle sorte que le composé organophosphoré de formule II peut être, par exemple, un phosphonate, un phosphinate, un oxyde de phosphine ou un phosphonamide, avec un composé carbonylé de formule III

$$Ar—CHO \qquad (III)$$

dans laquelle Ar est tel que défini pour la formule 1 pour conduire à un composé de formule IV

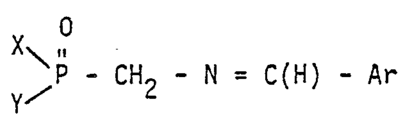

(IV)

dans laquelle les différents radicaux ont la signification fournie précédemment.

b) le composé de formule IV est traité avec une base de formule $B^{\ominus}M^{\oplus}$ pour conduire à un carbanion de formule V :

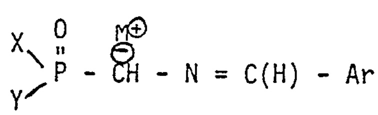

(V)

c) qui se transforme sous l'action de la chaleur en dérivé VI :

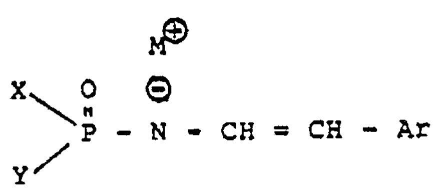

(VI)

pour aboutir après reprise par l'eau au composé de formule VII :

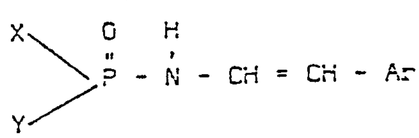

(VII)

Cette réaction est conduite généralement à une température comprise entre − 78 °C et + 150 °C choisie plus spécifiquement en fonction de la base $B^{\ominus}M^{\oplus}$ dans une plage plutôt haut de gamme en particulier lors de la réalisation de la phase c.

d) le composé de formule (VII) est transformé, sous l'action d'un agent réducteur en composé de formule VIII :

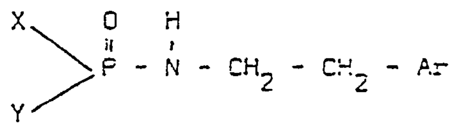

(VIII)

e) qui est finalement soumis à l'action d'un agent acide pour conduire au dérivé de formule 1 tel que défini plus haut.

Le procédé de préparation suivant l'invention peut être illustré par le schéma réactionnel suivant :

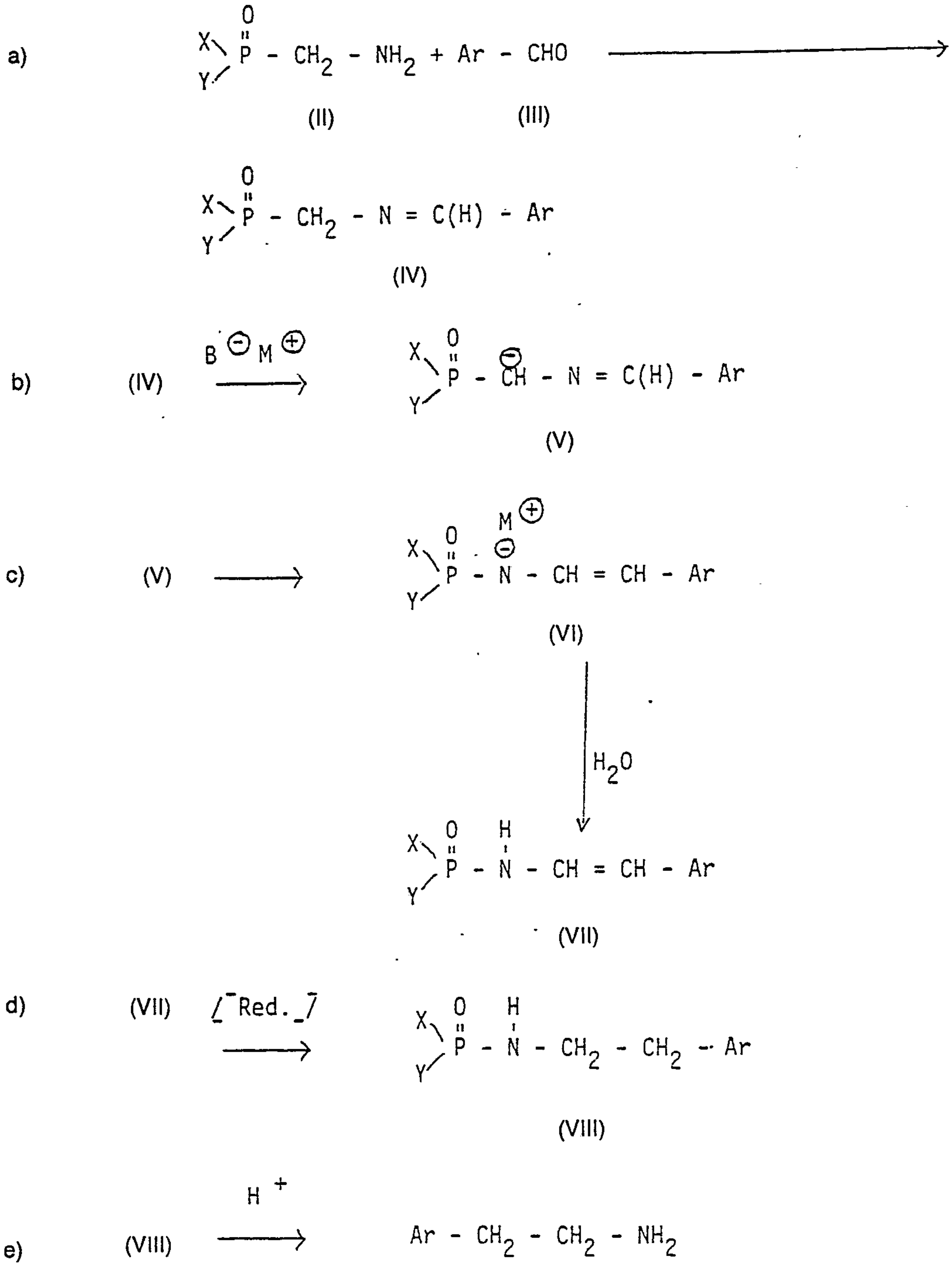


a) (II) (III) (IV)

b) (IV) (V)

c) (V) (VI) (VII)

d) (VII) (VIII)

e) (VIII) (I)

Le procédé peut avantageusement être mis en œuvre comme suit :

a) Les composés organophosphorés de formule 11, facilement accessibles par des procédés d'obtention bien connus tel que celui par exemple décrit par I. C Popoff et Coll. (J. Org. Chem. *28* 2898 (1963)) peuvent être mis à réagir avec les dérivés carbonylés 111 en l'absence de solvant et de catalyseur, l'eau formée au cours de la réaction étant éliminée à la fin de l'opération par des moyens appropriés. La condensation peut s'effectuer avantageusement dans un solvant tel qu'un hydrocarbure aromatique par exemple le toluène ou un alcool par exemple l'éthanol dans lesquels il est possible d'éliminer l'eau par distillation azéotropique. La condensation peut encore être avantageusement réalisée (sur le plan de la vitesse) en présence de quantités catalytiques d'un acide minéral ou organique comme par exemple l'acide paratoluènesulfonique. La température à laquelle on effectue cette transformation est variable mais se situe très généralement entre 20° et 120 °C.

b) La base $B^{\ominus}M^{\oplus}$ mise en œuvre dans ce stade peut être un hydrure de métal alcalin, notamment les hydrures de sodium, de lithium ou de potassium, un amidure ou alcoylamidure, notamment dialcoylamidure, de métal alcalin tel que le diisopropylamidure de lithium, un composé organométallique, notamment les organolithiens tels que le n-butyllithium ou les organosodés ou les organomagnésiens. On peut aussi faire appel aux alcoolates de métal alcalin ou alcalino-terreux, tels que le méthylate de sodium, de lithium, de potassium, de magnésium, le tertiobutylate de potassium, le tertioamylate de sodium. On peut encore utiliser des hydroxydes de métal alcalin ou alcalino-terreux, tels que l'hydroxyde de sodium, de lithium, de potassium, de magnésium.

En général, on utilise un équivalent stœchiométrique de la base $B^{\ominus}M^{\oplus}$ voire un léger excès par exemple de 10 % par rapport à l'équivalence. Mais il est également possible de mettre en œuvre des quantités de base inférieure, voire nettement inférieures à l'équivalence stœchiométrique. On opère généralement entre − 78 °C et + 150 °C à une température choisie plus spécifiquement en fonction de la base $B^-M^+$ dans une plage plutôt haut de gamme en particulier lors de la réalisation de la phase c. Les solvants préférés sont les éthers linéaires ou cycliques tel que le tétrahydrofurane, les hydrocarbures, notamment les aromatiques tels que benzène, toluène, xylènes, les alcools, les amides notamment le diméthylformamide, les sulfoxides notamment le diméthylsulfoxide. Il peut être également avantageux, particulièrement quand on utilise des hydroxides métalliques, d'opérer en système biphasique (eau + solvant tel que solvant halogéné comme par exemple le dichlorométhane, ou hydrocarbure aromatique tels que benzène, toluène, xylènes) en présence d'un catalyseur de transfert de phase notamment un sel d'ammonium quaternaire tel que l'iodure de tétra-n-butylammonium ou un sel de phosphonium. Les traitements habituels permettent d'isoler le composé V.

c) La réduction du dérivé de formule V est effectuée avantageusement par un hydrure mixte de métal alcalin comme notamment un borohydrure tel que le corohydrure de sodium ou le borohydrure de potassium. La réduction est réalisée au sein d'un solvant inerte comme un éther tel que par exemple le tétrahydrofurane ou le dioxane ou encore dans un alcool tel que par exemple le méthanol ou l'éthanol.

On peut également effectuer cette réduction par le biais d'une hydrogénation catalytique en phase homogène ou hétérogène dans des conditions bien connues d'une manière générale.

d) La coupure acido-catalysée de la liaison phosphore-azote du dérivé VI peut être réalisée en mettant en œuvre un acide minéral tel qu'un acide halohydrique comme par exemple l'acide chlorhydrique ou l'acide bromhydrique, mais également un acide organique en particulier un acide fort tel qu'un acide sulfonique comme par exemple l'acide benzènesulfonique ou l'acide paratoluènesulfonique. Les solvants préférés sont les éthers en particulier les éthers cycliques comme le tétrahydrofurane ou le dioxanne, les alcools comme le méthanol ou l'éthanol, les amides notamment le diméthylformamide, les sulfoxides notamment le diméthylsulfoxide. Il est possible de travailler au sein de ces solvants en l'absence d'eau mais aussi dans des mélanges contenant des proportions variables de cette dernière. Il est enfin possible de travailler au sein de l'eau elle-même.

En général, on utilise un à deux équivalents stœchiométriques d'acide. On opère généralement entre 0 et 100 °C et plus particulièrement entre 30 et 70 °C. Les composés de formule I ainsi obtenus peuvent ensuite être isolés et purifiés selon les méthodes habituelles. Pour réaliser ces opérations il peut être avantageux de transformer les composés libres de formule I en leurs sels, par exemple en leurs sels d'addition d'acides par réaction avec des acides minéraux ou organiques. A partir des sels, on peut libérer les composés de formule I selon les méthodes connues.

Les composés de formule IV :

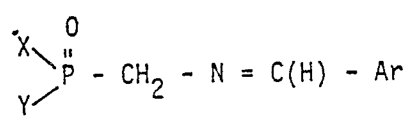

(IV)

Les composés de formule VII :

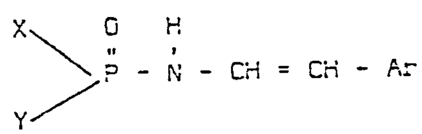

(VII)

Les composés de formule VIII :

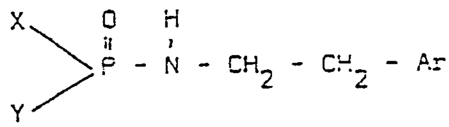 (VIII)

Les composés de formule I :

$$Ar—CH_2—CH_2—NH_2 \quad (I)$$

Les exemples suivants illustrent l'invention sans la limiter.

Exemple 1 — Préparation du chlorhydrate de la (thiényl-2)-2, éthylamine

Stade a — N-(thiénylidène-2), aminométhylphosphonate de diéthyl

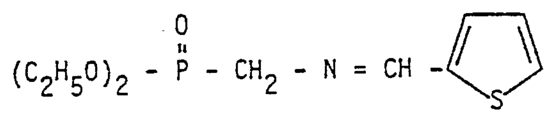

A 16,7 g (0,1 mole) d'aminométhylphosphonate de diéthyle dans 200 ml d'éthanol absolu, on ajoute 11,2 g (0,1 mole) de thénaldéhyde-2 et on porte 30 mn au reflux. L'eau formée au cours de la réaction est éliminée par distillation azéotropique. Après évaporation complète du solvant, on recueille 28 g (env. 100 %) d'une huile jaune pure par (CPL, CCM et CPG).

| | | |
|---|---|---|
| IR (film) | C=N | 1 645 $cm^{-1}$ |
| | P—O | 1 260 $cm^{-1}$ |
| | P—O—C | 1 060-1 080 $cm^{-1}$ |

| | | |
|---|---|---|
| RMN ($CDCl_3$) δ/TMS | 1,3 ppm | (t, 6H) |
| | 3,9 à 4,45 ppm | (m, 6H) |
| | 7 à 7,6 ppm | (m, 3H) |
| | 8,5 ppm | (d, 1H) |

Stades b, c — Bêta-(thiényl-2), N-vinylphosphoramidate de diéthyle

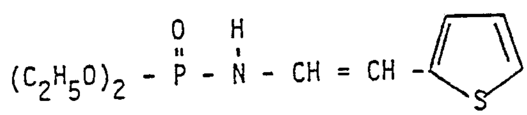

A une suspension de 11,2 g (0,1 mole) de terbutylate de potassium dans 160 ml de THF *, on ajoute goutte à goutte une solution de 27,9 g (0,1 mole) de N-(thiénylidène-2) aminométhylphosphonate de diéthyle dans 40 ml de THF. Au cours de l'addition, la température s'élève de 20° à 35 °C. A la fin de l'addition, on porte la température à 40-45 °C pendant 30' puis le milieu réactionnel est versé sur 400 ml d'une solution aqueuse saturée de chlorure d'ammonium. La phase aqueuse est extraite à l'éther isopropylique puis les phases éthérées réunies sont lavées avec une solution saturée de chlorure de sodium, séchées sur sulfate de sodium et évaporées pour fournir 20,9 g (75 %) de produit référencé sous forme d'une huile jaune.

| | | |
|---|---|---|
| RMN ($CDCl_3$) δ/TMS | 1,3 ppm | (t, 6H) |
| | 3,95 ppm | (ddeq, 4H) |
| | 6,35 ppm | (m, 1H) |
| | 6,9 à 7,5 ppm | (m, 5H) après échange avec $D_2O$ (m, 4H) |

| | | |
|---|---|---|
| IR (film) | NH | 3 300 $cm^{-1}$ |
| | C = C | 1 645 $cm^{-1}$ |
| | P—O | 1 250 $cm^{-1}$ |
| | P—O—C | 1 050 $cm^{-1}$ |

*THF = TETRAHYDROFURANE

Stade d — N-[(thiényl-2)-2 éthyl], phosphoramidate de diéthyle

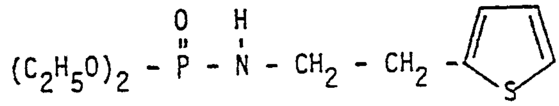

Les 20,9 g (0,075 mole) de beta-(thiényl-2), N-vinylphosphoramidade de diéthyle obtenus ci-dessus sont additionnés à une solution de 5,1 g (0,075 mole) de borohydrure de sodium dans 200 ml d'éthanol.

Durant l'addition, la température s'élève puis se stabilise à 30 °C. Après 2 heures d'agitation supplémentaires, la température du milieu est portée à 45-50 °C pendant une heure, puis l'éthanol est évaporé et le résidu repris par un mélange d'éther isopropylique et d'eau.

La phase aqueuse est réextraite plusieurs fois à l'éther isopropylique et les phases organiques réunies sont lavées à l'eau, séchées sur sulfate de sodium et évaporées pour fournir 21 g (env. 75 % par rapport à l'aminométhylphosphonate de départ) de produit référencé sous forme d'une huile jaune.

IR (film)
- 3 400 $cm^{-1}$
- 1 520 $cm^{-1}$
- 1 275 $cm^{-1}$
- 1 210 $cm^{-1}$

RMN ($CDCl_3$) δ/TMS
- 1,3 ppm (t, 6H)
- 3,1 ppm (m, 5H) Ar—$CH_2$ $CH_2$—NH (par échange avec $D_2O$, on obtient à 3,1 ppm (m, 4H)
- 4,05 ppm (ddeq, 4H)
- 6,75 à 7,2 ppm (m, 3H)

Stade e — Chlorhydrate de (thiényl-2)-2 éthylamine

Un mélange bien agité de 21 g de phosphoramidate obtenu ci-dessus et de 100 ml d'une solution aqueuse 6N d'acide chlorhydrique est porté à 80-85 °C durant 1 h 30. Après refroidissement et extraction du milieu réactionnel avec 30 ml de chlorure de méthylène, la phase aqueuse isolée est basifiée avec une solution aqueuse de soude puis extraite avec de l'éther isopropylique.

La phase éthérée, isolée puis séchée sur sulfate de sodium est additionnée par barbotage d'acide chlorhydrique gazeux qui provoque la précipitation de cristaux que l'on filtre et purifie par dissolution dans l'éthanol puis reprécipitation par addition d'éther isopropylique. On obtient ainsi 8,9 g (rendement global : 54 %) de chlorhydrate de (thiényl-2)-2 éthylamine sous forme de cristaux blancs.

F = 202 °C

RMN ($D_2O$)
- 3,25 ppm (s, 4H)
- 7 ppm (m, 2H)
- 7,35 ppm (m, 1H) Pic de l'eau à 4,65 ppm

IR (pastille KBr)
- 3 000 $cm^{-1}$
- 1 590 $cm^{-1}$
- 1 470 $cm^{-1}$
- 1 230 $cm^{-1}$

Analyse $C_6H_{10}NS$, HCl = 164,679
calculé C 43,75 H 6,73 N 8,50
trouvé C 43,70 H 6,77 N 8,45

Exemple n° 2 — Préparation du chlorhydrate de phényl-2 éthylamine

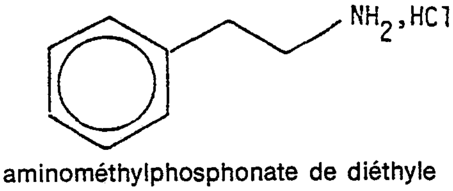

Stade a — N-benzylidène aminométhylphosphonate de diéthyle

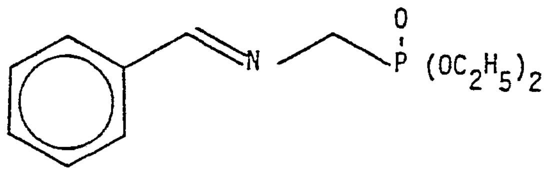

L'imine de référence est préparée avec un rendement de 100 % en faisant réagir 0,1 mole (16,7 g) d'aminométhylphosphonate de diéthyle avec 10,6 g (0,1 mole) de benzaldéhyde dans les mêmes conditions que celles décrites à l'exemple 1.'

Le produit obtenu est une huile jaune claire.

| | | | |
|---|---|---|---|
| RMN ($CDCl_3$) δ/TMS | 1,3 ppm | (t, 6H) |
| | 4 ppm | (m, 6H) |
| | 7,2 à 7,8 ppm | (m, 5H) |
| | 8,2 ppm | (d, 1H) |

| | | |
|---|---|---|
| IR (film) | C = N | 1 640 $cm^{-1}$ |
| | P—O | 1 250 $cm^{-1}$ |

Stades b, c — Bêta-phényl, N-vinyl, phosphoramidate de diéthyle

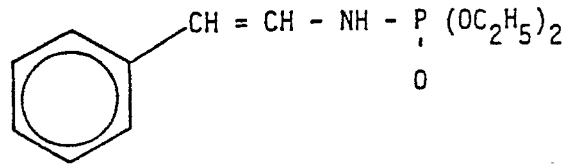

A une solution de 25,5 g (0,1 mole) de N-benzylidène, aminométhyl-phosphonate de diéthyle dans 200 ml de THF, maintenue à 20 °C, on ajoute goutte à goutte 35,7 ml (0,1 mole) d'une solution 2,8 M de n-Butyllithium dans le cyclohexane. A la fin de l'addition le milieu réactionnel est porté à 35 °C, maintenu à cette température, pendant 30 mn puis versé dans 1 litre d'une solution aqueuse saturée de chlorure d'ammonium et enfin extrait à l'éther isopropylique.

La phase éthérée lavée à l'eau, séchée sur sulfate de sodium et évaporée, fournit une huile jaune qui se concrétise par trituration dans l'hexane.

Après filtration et recristallisation dans l'hexane les cristaux obtenus sont lavés avec un mélange hexane-éther isopropylique (90/10) puis séchés sous vide à température ambiante. On obtient ainsi 17,9 g (70 %) de béta-phényl, N-vinyl, phosphoramidate de diéthyle.

F = 60 °C

| | | |
|---|---|---|
| IR (pastille KBr) | NH | 3 400 $cm^{-1}$ |
| | CH = CH | 1 650 $cm^{-1}$ |
| | P—O | 1 250 $cm^{-1}$ |

| | | | |
|---|---|---|---|
| RMN ($CDCl_3$) δ/TMS | 1,3 ppm | (t, 6H) | |
| | 4,05 ppm | (ddeq, 4H) | |
| | * 5,85 ppm | (dded, 1H) | $J_1$ = 13 Hz |
| | ** 6,65 ppm | (m, 2H) | $J_2$ = 6 Hz |
| | 7,15 ppm | (s, 5H) | |

| | | | |
|---|---|---|---|
| après échange avec $D_2O$ S : | * 5,85 (d, 1H) | J = 13 Hz |
| | ** 6,65 (dded, 1H) | $J_1$ = 13 Hz |
| | | $J_2$ = 16 Hz |

Analyse : $C_{12}H_{18}NO_3P$ = 255,243
Calculé : C % 56,46 H % 7,10 N % 5,48
Trouvé : C % 56,10 H % 7,20 N % 5,50

Stade d — N-(phényl-2 éthyl), phosphoramidate de diéthyle

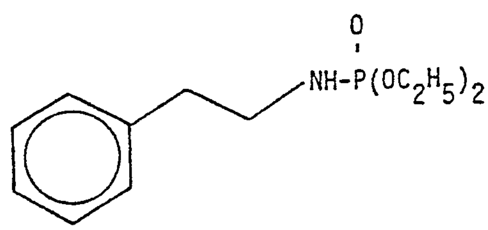

12,75 g (0,05 mole) du N-vinyl, phosphoramidate préparé aux stades b, c, sont réduits par le borohydrure de sodium dans l'éthanol dans les conditions décrites à l'exemple I. On obtient ainsi 12,85 g (rendement de la réduction : 100 %) du phosphoramidate de référence sous forme d'une huile jaune clair.

IR (film)

| | |
|---|---|
| IR (film) | 3 200 $cm^{-1}$ |
| | 2 900 $cm^{-1}$ |
| | 1 475 $cm^{-1}$ |
| | 1 275 $cm^{-1}$ |
| | 1 220 $cm^{-1}$ |

| | | |
|---|---|---|
| RMN ($CDCl_3$) δ/TMS | 1,33 ppm | (t, 6H) |
| | env. 3 ppm | (m, 5H) dont 1H échangeable avec $D_2O$ |
| | 4 ppm | (q, d 4H) |
| | 7,2 ppm | (s, 5H) |

Stade e — Chlorhydrate de phényl-2 éthylamine

12,85 g (0,05 mole) du phosphoramidate préparé ci-dessus sont traités par une solution aqueuse d'acide chlorhydrique 6N dans les conditions décrites à l'exemple 1.

L'amine est isolée puis chlorhydratée dans l'éther isopropylique. On obtient ainsi 7,5 g de chlorhydrate de phényl-2 éthylamine sous forme de cristaux blancs (rendement 95 % par rapport au phosphoramidate précurseur et rendement global à partir de l'aminométhylphosphonate d'éthyl 66,5 %).

F = 222 °C

| | | |
|---|---|---|
| IR (film, sur la base) | 3 400 $cm^{-1}$ | |
| | 3 000 $cm^{-1}$ | 2 900 $cm^{-1}$ |
| | 1 600 $cm^{-1}$ | |
| | 1 490 $cm^{-1}$ | 1 450 $cm^{-1}$ |
| | 820 $cm^{-1}$ | |
| | 725 $cm^{-1}$ | 700 $cm^{-1}$ |

| | | |
|---|---|---|
| RMN ($CDCl_3$ sur la base) δ/TMS | 2,8 ppm | (m, 4H) |
| | 7,2 ppm | (s, 5H) |
| | 1,1 ppm | (s, 2H) échangeable avec $D_2O$ |

Analyse $C_8H_{11}$, HCl = 157,643
Calculé : C % 60,94 H % 7,67 N % 8,88
Trouvé : C % 61,01 H % 7,70 N % 8,85

Exemple n° 3 — Préparation du chlorhydrate de phényl-2 éthylamine

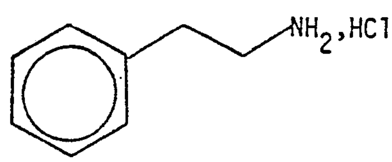

Stade a — N-benzylidène aminométhylphosphonate d'isopropyle

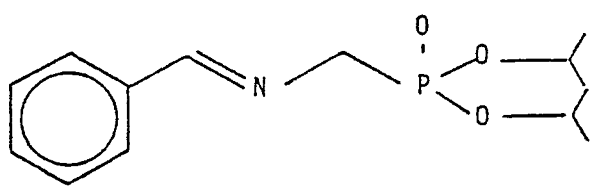

L'imine de référence est préparée quantitativement par action de 10,6 g (0,1 mole) de benzaldéhyde sur 19,5 g (0,1 mole) d'aminométhyl-phosphonate d'isopropyle dans les conditions décrites à l'exemple 1.

| | | |
|---|---|---|
| IR (film) | C = N | 1 640 $cm^{-1}$ |
| | P—O | 1 250 $cm^{-1}$ |

| | | |
|---|---|---|
| RMN ($CDCl_3$) δ/TMS | 1,35 ppm | (d, 12H) |
| | 3,95 ppm | (d, 2H) |
| | 4,5 ppm | (m, 2H) |
| | 7,2 à 7,8 ppm | (m, 5H) |
| | 8,2 ppm | (d, 1H) |

Stades b, c — Béta-phényl, N-vinyl, phosphoramidate de diisopropyl

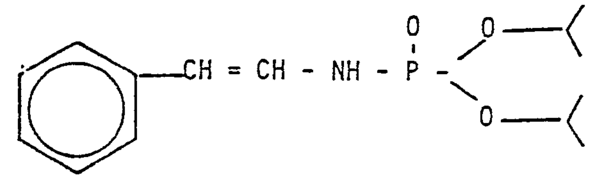

A une solution de 28,3 g (0,1 mole) de N-benzylidène aminométhyl-phosphonate de diisopropyle dans 200 ml de THF, on ajoute à 20 °C, goutte à goutte 35,7 ml (0,1 mole) d'une solution 2,8 M de n-butyl lithium dans l'hexane. Après la fin de l'addition, on porte le milieu réactionnel à 35 °C pendant 30′ puis on hydrolyse et extrait la phase organique avec de l'éther isopropylique.

La phase éthérée est séchée sur sulfate de sodium puis évaporée. L'huile jaune obtenue est triturée dans l'hexane pour fournir 21,2 g (rendement 75 %) d'un solide jaune.

F = 98 °C

RMN ($CDCl_3$)
- 1,35 ppm (d, 12H)
- 4,5 ppm (m, 2H)
- * 5,80 ppm (dded, 1H) $J_1$ = 13 Hz, $J_2$ = 6 Hz
- ** 6,65 ppm (m, 2H)
- 7,15 ppm (s, 5H)

après échange avec $D_2O$ on a :
- * 5,80 ppm (d, 1H)
- ** 6,65 ppm (d,d 1H) $J_1$ = 13 Hz, $J_2$ = 15 Hz

IR (pastille KBr)
- 3 400 $cm^{-1}$
- 1 650 $cm^{-1}$
- 1 250 $cm^{-1}$

Analyse $C_{14}H_{22}NO_3P$ = 283,303

| | | | |
|---|---|---|---|
| Calculé : | C % 59,34 | H % 7,82 | N % 4,94 |
| Trouvé : | C % 59,25 | H % 7,80 | N % 5,00 |

Stade d — N(phényl-2 éthyl), phosphoramidate d'isopropyle

1,6 g du N-vinyl, phosphoramidate préparé ci-dessus en solution dans 80 ml de dioxanne sont hydrogénés en 6 heures en présence de 160 mg de palladium à 10 % sur charbon sous une pression de 3 Bars et à une température comprise entre 55 et 60 °C. Après filtration du catalyseur et évaporation du dioxanne, on recueille 1,6 g (env. 100 %) du phosphoramidate de référence sous forme d'une huile incolore.

RMN ($CDCl_3$) δ/TMS
- 1,35 ppm (d, 12H)
- * 3 ppm (m, 5H)
- 4,5 ppm (m, 2H)
- 7,2 ppm (s, 5H)

* après échange avec $D_2O$ on a à 3 ppm (m, 4H)

IR (film)
- 3 250 $cm^{-1}$
- 2 900 $cm^{-1}$
- 1 475 $cm^{-1}$
- 1 275 $cm^{-1}$
- 1 220 $cm^{-1}$

Stade e — Chlorhydrate de phényl-2, éthylamine

Une solution de 1,425 g (5 m mole) de N(phényl-2 éthyl), phosphoramidate d'isopropyle dans 20 ml d'éther isopropylique saturé en acide chlorhydrique gazeux est agitée une nuit à température ambiante. Le précipité de chlorhydrate de phényl-2, éthylamine formé est filtré, lavé à l'éther isopropylique puis séché sous vide.

On obtient ainsi 0,75 g (rendement 95 %) d'un solide cristallisé dont les caractéristiques physiques et spectrales sont identiques à celle du composé préparé à l'exemple 2.

Exemple 4 — Préparation du chlorhydrate de la (bromo-5, thiényl-2)-2 éthylamine

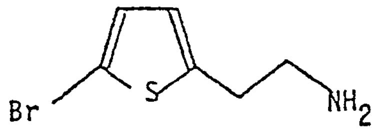

, HCl

Stade a — N-(bromo-5, thiénylidène-2) aminoéthylphosphonate de diéthyle

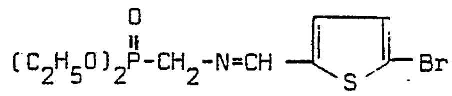

A partir de 16,7 g (0,1 mole) d'aminométhylphosphonate de diéthyle et de 19,1 g (0,1 mole) de bromo-5, thénaldéhyde-2 on obtient, en opérant de façon identique à celle décrite à l'exemple 1, 34 g (rendement 100 %) du produit référencé sous forme d'une huile jaune.

IR (film)
- 3 000 $cm^{-1}$
- 2 900 $cm^{-1}$
- 1 630 $cm^{-1}$
- 1 430 $cm^{-1}$
- 1 250 $cm^{-1}$
- 1 050 $cm^{-1}$

RMN ($CDCl_3$) δ/TMS

| | | |
|---|---|---|
| | 1,3 ppm | (t, 6H) |
| | 4,1 ppm | (m, 6H) |
| | 7 à 7,5 ppm | (m, 2H) |
| | 8,2 ppm | (d, 1H) |

Stades b, c — (Béta-(bromo-5, thiényle-2), N-vinyl, phosphoramidate de diéthyle

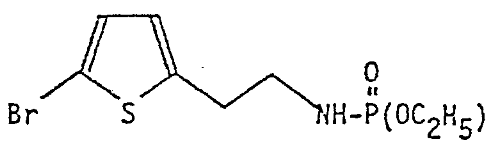

34 g (0,1 mole) de l'imine préparée ci-dessus et de terbutylate de potassium fournissent dans les conditions décrites à l'exemple 1, 30 g du produit référencé, sous forme d'une huile jaune orangée que l'on utilise telle quelle dans l'étape suivante.

Stade d — N-[(bromo-5, thiényl-2)-2 éthyl], phosphoramidate de diéthyle

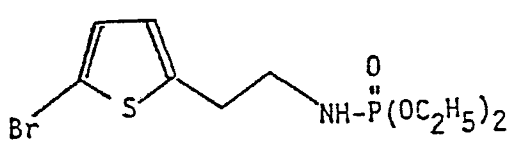

Le vinylphosphoramidate brut obtenu aux stades b, c traité par 3,8 g (0,1 mole) de borohydrure de sodium dans les conditions décrites à l'exemple 1 fournit le N-[(bromo-5, thiényl-2)-2 éthyl] phosphoramidate de diéthyle brut qui est purifié par chromatographie sur colonne de silice (éluant : acétate d'éthyle) on obtient ainsi 17,1 g de phosphoramidate référencé sous forme d'une huile jaune (50 % par rapport à l'aminométhylphosphonate d'éthyle engagé au stade a).

IR (film)
- 3 400 $cm^{-1}$
- 3 250 $cm^{-1}$
- 3 000 à 2 850 $cm^{-1}$
- 1 450 $cm^{-1}$
- 1 240 $cm^{-1}$
- 1 040 $cm^{-1}$

RMN ($CDCl_3$) δ/TMS
- 1,33 ppm (t, 6H)
- 3 ppm (m, 5H) (*)
- 4 ppm (dq, 4H)
- 6,55 ppm (d, 1H) } système AB avec $J_{AB}$ = 4 Hz
- 6,80 ppm (d, 1H) }

Stade e — Chlorhydate de la (bromo-5, thiényl-2)-2 éthylamine

Une solution contenant 3,42 g (0,01 mole) de N-[(bromo-5, thiényl-2)-2 éthyl] phosphoramidate de diéthyle dans 50 ml d'éther isopropylique saturé d'acide chlorhydrique gazeux est agité durant une nuit à température ambiante. Les cristaux formés sont filtrés et lavés avec de l'éther isopropylique. On obtient ainsi, après séchage, 1,45 g (rendement 60 % par rapport au phosphoramidate mis en œuvre) du produit référencé sous forme de paillettes gris argenté.

F = 220 °C (déc.)

IR (pastille KBr)
- 3 400 $cm^{-1}$
- 3 000 $cm^{-1}$
- 1 600 $cm^{-1}$
- 1 450 $cm^{-1}$
- 1 170-1 150 $cm^{-1}$

RMN (DMSO $d_6$) δ/TMS
- 3,05 ppm (s large, 3 ppm)
- 6,65 ppm (d, 1H) } système AB avec $J_{AB}$ = 4 Hz
- 6,98 ppm (d, 1H) }
- 8,4 ppm (3H) échangeable avec $D_2O$

Analyse $C_6H_8BrNS$, HCl = 242,58
Calculé : C % 29,70 H % 3,73 N % 5,77
Trouvé : C % 29,71 H % 3,73 N % 5,72

Exemple 5 — Préparation du chlorhydrate de la (naphtyl-1)-2 éthylamine

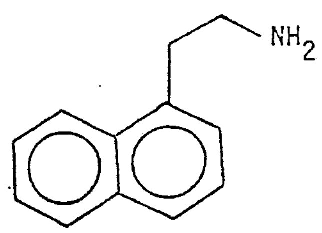

, HCl

Stade a — Imine du naphtyl-1 carboxaldéhyde et de l'aminométhylphosphonate d'éthyle

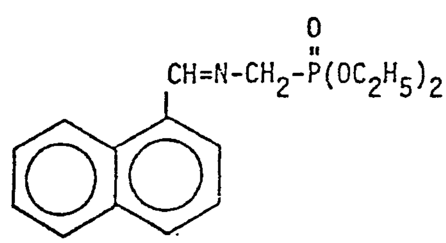

A partir de 15,6 g (0,1 mole) de naphtalène-1 carboxaldéhyde et de 16,7 g (0,1 mole) d'aminométhylphosphonate d'éthyle on prépare 30,5 g (rendement 100 %) du produit référencé par un mode opératoire identique à celui décrit à l'exemple 1.

IR (film)
- 3 000 $cm^{-1}$
- 1 640 $cm^{-1}$
- 1 510 $cm^{-1}$
- 1 250 $cm^{-1}$
- 1 030-1 050 $cm^{-1}$

(*) par échange avec $D_2O$ on a (m, 4H)

| | | | |
|---|---|---|---|
| RMN ($CDCl_3$) | δ/TMS | 1,3 ppm | (t, 6H) |
| | | 4 ppm | (m, 6H) |
| | | 6,9 à 8,1 ppm | (m, 7H) |
| | | 8,3 ppm | (d, 1H) |

Stades b, c — Béta-naphtyl-1, N-vinyl, phosphoramidate de diéthyle

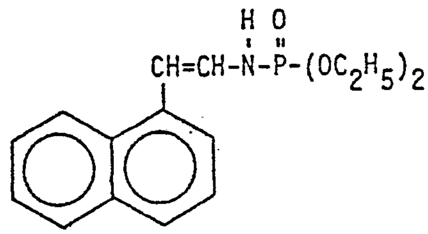

A partir de 30,5 g (0,1 mole) de l'imine préparée au stade précédent et de 11,2 g (0,1 mole) de terbutylate de potassium on obtient en utilisant le mode opératoire décrit à l'exemple 1, 22,3 g (rendement : 73 %) du produit référencé, sous forme d'une huile jaune clair.

| | |
|---|---|
| IR (film) | 3 400 $cm^{-1}$ |
| | 3 200 $cm^{-1}$ |
| | 1 650 $cm^{-1}$ |
| | 1 250 $cm^{-1}$ |
| | 1 050 $cm^{-1}$ |

| | | | |
|---|---|---|---|
| RMN ($CDCl_3$) | δ/TMS | 1,3 ppm | (t, 6H) |
| | | 4,1 ppm | (qd, 4H) |
| | | 6,7 à 6,9 ppm | (m, 2H) |
| | | 7 à 8,1 ppm | (m, 8H) |

Par addition de $D_2O$ le spectre se simplifie un peu et l'intégration diminue.

Stade d — N[(naphtyl-1)-2 éthyl] phosphoramidate de diéthyle

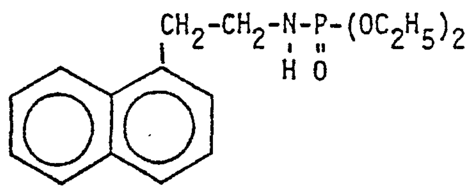

A partir des 22,3 g (0,073 mole) du vinyl, phosphoramidate obtenu ci-dessus et de 2,85 g (0,075 mole) de borohydrure de sodium, on prépare selon un mode opératoire identique à celui décrit à l'exemple 1, 22 g du produit référencé, (rendement : 71,6 % par rapport à l'aminométhylphosphonate d'éthyle) sous forme d'une huile jaune clair.

| | |
|---|---|
| IR (film) | 3 400-3 240 $cm^{-1}$ |
| | 3 000 $cm^{-1}$ |
| | 2 900 $cm^{-1}$ |
| | 1 600 $cm^{-1}$ |
| | 1 510 $cm^{-1}$ |
| | 1 240-1 035 $cm^{-1}$ |

| | | | |
|---|---|---|---|
| RMN ($CDCl_3$) | δ/TMS | 1,3 ppm | (t, 6H) |
| | | 3,2 ppm | (m, 5H) qui donne après échange avec $CD_3OD$ (m, 4H) |
| | | 3,95 ppm | (qd, 4H) |
| | | 7,2 à 8 ppm | (m, 7H) |

Stade e — Chlorhydrate de la (naphtyl-1)-2 éthylamine

En opérant comme décrit à l'exemple 1 sur les 22 g de phosphoramidate obtenu ci-dessus on obtient 11,6 g (rendement 61 % par rapport à l'aminométhylphosphonate d'éthyle) du chlorhydrate référencé sous forme de cristaux blancs.

F = 260 °C (déc.)

IR (pastille KBr) { 3 400 $cm^{-1}$, 3 050 $cm^{-1}$, 1 600 $cm^{-1}$, 1 510 $cm^{-1}$, 1 495 $cm^{-1}$, 1 400 $cm^{-1}$, 800 $cm^{-1}$, 775 $cm^{-1}$

| RMN (DMSO $d_6$) δ/TMS | | |
|---|---|---|
| | 3,4 ppm | (m, 4H) |
| | 7,3 à 8,3 ppm | (m, 7H) |
| | 8,65 ppm | (m, 3H) échangeable avec $D_2O$ |

Analyse $C_{12}H_{13}N$, Hcl = 207,69
Calculé : C % 69,39 H % 6,79 N % 6,74
Trouvé : C % 69,44 H % 6,76 N % 6,54

Exemple 6 — Préparation du chlorhydrate de la paraméthoxyphényl-2 éthylamine

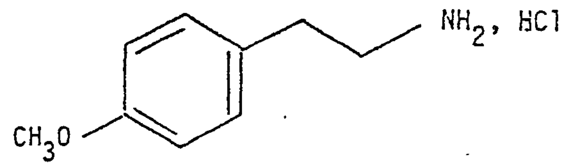

Stade a — N-paramethoxybenzylidène, aminométhylphosphonate de diéthyle

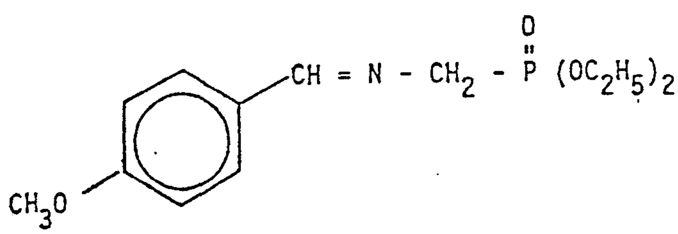

A partir de 16,7 g (0,1 mole) d'aminométhylphosphonate d'éthyle et de 13,6 g (0,1 mole) de paraméthoxybenzaldéhyde, on prépare selon le procédé décrit à l'exemple 1, 28,5 g (rendement 100 %) du produit référencé, sous forme d'une huile jaune.

IR (film) { 1 630 $cm^{-1}$, 1 250 $cm^{-1}$, 1 040 $cm^{-1}$

| RMN ($CDCl_3$) δ/TMS | | | |
|---|---|---|---|
| | 1,3 ppm | (t, 6H) | |
| | 3,75 ppm | (s, 3H) | |
| | 4,15 ppm | (m, 6H) | |
| | 6,90 ppm | (d, 2H) | système $A_2B_2$ |
| | 7,75 ppm | (d, 2H) | système $A_2B_2$ |
| | 8,30 ppm | (d, 1H) | |

Stades b, c — Bêta-paraméthoxyphényl, N-vinyl, phosphoramidate de diéthyle

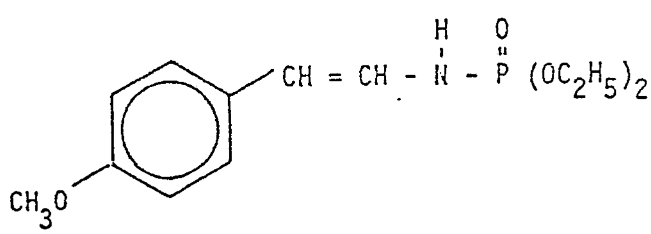

A partir de 28,5 g (0,1 mole) de l'imine préparée au stade précédent et selon un procédé analogue à celui décrit à l'exemple 1, on obtient 20 g (rendement 70 %) du produit référencé sous forme d'une huile jaune que l'on utilise telle quelle dans l'étape suivante.

Stade d — N-[(paraméthoxybenzyl-2)-2 éthyl], phosphoramidate de diéthyle

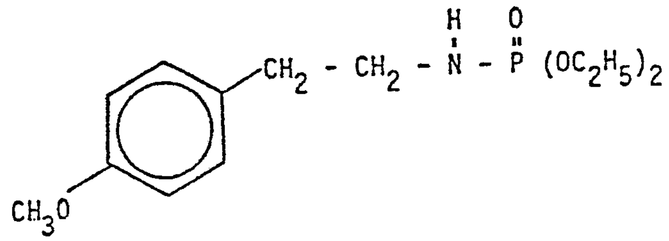

A partir de 20 g (0,07 mole) du vinylphosphoramidate préparé aux stades précédents et de 2,66 g (0,07 mole) de borohydrure de sodium et en opérant selon des conditions analogues à celles décrites à l'exemple 1, on obtient 20 g (rendement 69,5 % par rapport à l'aminométhyl phosphonate d'éthyle) du produit référencé sous forme d'une huile.

IR (film) { $3\,400\ cm^{-1}$ $3\,240\ cm^{-1}$
$1\,240\ cm^{-1}$
$1\,035\ cm^{-1}$

RMN ($CDCl_3$) δ/TMS
- 1,3 ppm (t, 6H)
- 3,2 ppm (m, 5H) (m,4H) après échange avec $CD_3OD$
- 3,75 ppm (s, 3H)
- 3,95 ppm (q,d 4H)
- 6,90 ppm (d, 2H) } système $A_2B_2$
- 7,2 ppm (d, 2H) }

Stade e — Chlorhydrate de la paraméthoxyphényl-2 éthylamine

En opérant comme décrit à l'exemple 1, sur le phosphoramidate obtenu ci-dessus, on obtient 8 g (53 % par rapport à l'aminométhylphosphonate de départ) de chlorhydrate référencé, sous forme de cristaux blancs.

F = 217 °C.

IR (sur la base en film)
- $3\,350\ cm^{-1}$
- $2\,950\ cm^{-1}$
- $1\,610\ cm^{-1}$
- $1\,510\ cm^{-1}$
- $1\,210\ cm^{-1}$

RMN ($D_2O$)
- 3,15 ppm (m, 4H)
- 3,8 ppm (s, 3H)
- 6,85 ppm (d, 2H) } système AB
- 7,3 ppm (d, 2H) }
- pic de l'eau à 4,75 ppm

Analyse : $C_9H_{13}NO$, HCl = 187,66
Calculé : C % 57,59 H % 7,51 N % 7,46
Trouvé : C % 57,55 H % 7,48 N % 7,50

Exemple 7 — Préparation de la (pyridyl-4)-2 éthylamine

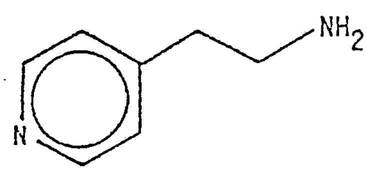

Stade a — Imine du pyridyl-4 carboxaldéhyde et de l'aminométhylphosphonate d'éthyle

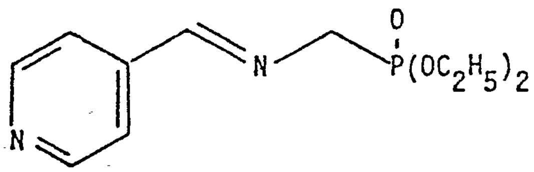

A partir de 10,7 g (0,1 mole) de pyridyl-4 carboxaldéhyde et de 16,7 g (0,1 mole) d'aminométhylphosphonate de diéthyle on prépare selon le procédé décrit à l'exemple 1, 25,6 g (100 %) du produit référencé, sous forme d'une huile orangée.

| | |
|---|---|
| IR (film) | 1 635 $cm^{-1}$ |
| | 1 250 $cm^{-1}$ |
| | 1 045 $cm^{-1}$ |

| | | | | |
|---|---|---|---|---|
| RMN ($CDCl_3$) δ/TMS | 1,3 ppm | (j, 3H) | | |
| | 4,10 ppm | (m, 6H) | | |
| | 7,7 ppm | (d, 2H) | | système $A_2B_2$ |
| | 8,4 ppm | (d, 1H) | N = CH—Ar | |
| | 8,75 ppm | (d, 2H) | | système $A_2B_2$ |

Stades b, c — Béta-pyridyl-4, N-vinyl, phosphoramidate d'éthyle

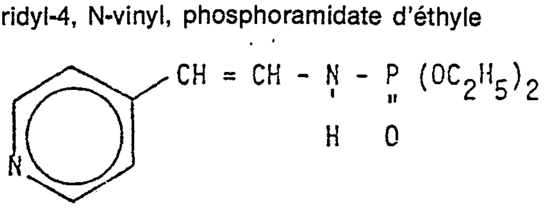

A partir de 25,6 g (0,1 mole) de l'imine préparée au stade a et en opérant de façon analogue à celle décrite à l'exemple 1, on isole un produit brut sous forme d'une huile orangée qui, après purification par chromatographie sur colonne de silice (éluant 90 % acétate d'éthyle, 10 % éthanol) fournit 13 g (rendement 50,7 %) du produit référencé sous forme de cristaux oranges.

F = 75 °C.

| | |
|---|---|
| IR (pastille KBr) | 3 400 $cm^{-1}$ |
| | 3 150 $cm^{-1}$ |
| | 1 900 $cm^{-1}$ |
| | 1 650 $cm^{-1}$ |
| | 1 600 $cm^{-1}$ |
| | 1 250-1 040 $cm^{-1}$ |

| | | | |
|---|---|---|---|
| RMN ($CDCl_3$ + $D_2O$) δ/TMS | 1,35 ppm | (t, 6H) | |
| | 4,15 ppm | (dp, 4H) | |
| | 5,80 ppm | (d, 1H) | $J_1$ = 15 Hz |
| | 7 ppm | (dd, 1H) | $J_1$ = 15 Hz<br>$J_2$ = 19 Hz |
| | | couplage H-P | |
| | 6,95 ppm | (d, 2H) | système $A_2B_2$ |
| | 8,25 ppm | (d, 2H) | système $A_2B_2$ |

Stade d — N-[(pyridyl-4)-2 éthyl] phosphoramidate d'éthyle

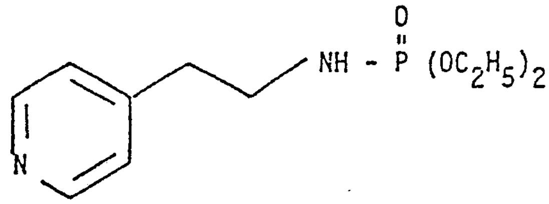

La réduction par le borohydrure de sodium du produit obtenu à l'étape précédente selon le procédé décrit à l'exemple 1 fournit 13 g (rendement 50,3 % par rapport à l'aminométhylphosphonate de départ) du produit référencé, sous forme d'une huile.

| | |
|---|---|
| IR (film) | 3 400-3 250 $cm^{-1}$<br>1 600 $cm^{-1}$<br>1 250 $cm^{-1}$<br>1 045 $cm^{-1}$ |

| | | | |
|---|---|---|---|
| RMN ($CDCl_3$) | 1,3 ppm | (t, 6H) | |
| | 3,2 ppm | (m, 5H) dont 1 échangeable avec $D_2O$ | |
| | 4,15 ppm | (qd, 4H) | |
| | 7,9 ppm | (d, 2H) | système $A_2B_2$ |
| | 8,65 ppm | (d, 2H) | |

Stade e — (pyridyl-4)-2 éthylamine

Le traitement du phosphoramidate obtenu au stade d par une solution aqueuse d'acide chlorhydrique dans les conditions décrites à l'exemple 1, fournit après basification, 5,6 g (rendement 47,5 % par rapport à l'aminométhylphosphonate de diéthyle) de l'amine référencée sous forme d'une huile jaune qui brunit à l'air.

| | |
|---|---|
| IR (film) | 3 300 $cm^{-1}$<br>2 900 $cm^{-1}$<br>1 600 $cm^{-1}$<br>1 440 $cm^{-1}$ |

| | | | |
|---|---|---|---|
| RMN ($DCl/D_2O$) | 3,3 ppm | (s, 4H) | |
| | 8 ppm | (d, 2H) | système $A_2B_2$ |
| | 8,7 ppm | (d, 2H) | |

Exemple 8 — Préparation du chlorhydrate de la (furyl-2)-2 éthylamine

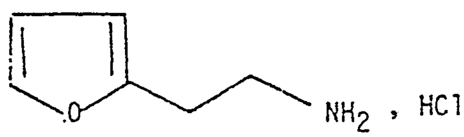

Stade a — N-furfurylidène-2, aminométhylphosphonate de diéthyle

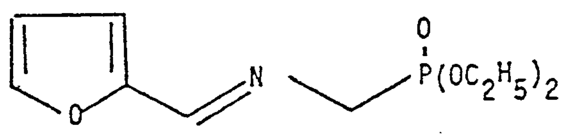

On prépare 0,1 mole de produit référencé (sous forme d'une huile jaune) en opérant comme décrit à l'exemple 1.

| | |
|---|---|
| IR (film) | 1 645 $cm^{-1}$<br>1 250 $cm^{-1}$<br>1 060 $cm^{-1}$<br>1 050 $cm^{-1}$ |

| | | |
|---|---|---|
| RMN ($CDCl_3$) δ/TMS | 1,3 ppm | (t, 6H) |
| | 4 ppm | (m, 6H) |
| | 7 à 7,5 ppm | (m, 3H) |
| | 8,3 ppm | (d, 1H) |

Stades b, c — Béta-furyl-2, N-vinyl, phosphoramidate de diéthyle

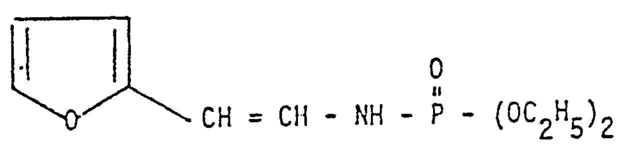

A partir de 0,1 mole de l'imine précédente et en opérant comme décrit à l'exemple 1, on prépare 18 g de produit référencé (sous forme d'une huile) que l'on utilise telle quelle au stade suivant.

Stade d — N-[(furyl-2)-2 éthyl], phosphoramidate d'éthyle

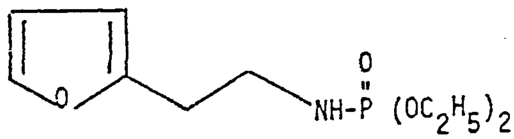

Le vinylphosphoramidate obtenu ci-dessus est réduit par le borohydrure de sodium selon le procédé décrit à l'exemple 1. Après purification par chromatographie sur colonne de silice (éluant : acétate d'éthyle), on obtient 14 g (rendement 57 % par rapport à l'aminométhylphosphonate) du produit référencé sous forme d'une huile jaune.

| | |
|---|---|
| IR (film) | 3 400 $cm^{-1}$ |
| | 3 250 $cm^{-1}$ |
| | 1 600 $cm^{-1}$ |
| | 1 510 $cm^{-1}$ |
| | 1 240 $cm^{-1}$ |
| | 1 060 $cm^{-1}$-1 030 $cm^{-1}$ |

| | | |
|---|---|---|
| RMN ($CDCl_3$) | 1,3 ppm | (t, 6H) |
| | 3 ppm | (m, 5H) (m, 4H) après échange avec $D_2O$ |
| | 4 ppm | (dp, 4H) |
| | 6 ppm | (d, 1H) |
| | 6,2 ppm | (dd, 1H) |
| | 7,2 ppm | (d, 1H) |

Stade e — Chlorhydrate de la (furyl-2)-2 éthylamine

Une solution du phosphoramidate obtenu au stade précédent dans 100 ml d'éther isopropylique saturé d'acide chlorhydrique gazeux est agité durant une nuit à température ambiante. Le précipité formé est isolé, redissous dans le minimum d'éthanol puis reprécipité par addition d'éther isopropylique. On obtient ainsi, après filtration et séchage sous vide, 6,7 g de chlorhydrate de (furyl-2)-2 éthylamine (rendement 45 % par rapport à l'aminométhylphosphonate d'éthyle) sous forme de cristaux blancs.

F = 204 °C

| | | |
|---|---|---|
| IR (pastille KBr) | 2 800 à 3 200 $cm^{-1}$ | |
| | 1 600 $cm^{-1}$ | 1 580 $cm^{-1}$ |
| | 1 500 $cm^{-1}$ | |
| | 1 210 $cm^{-1}$ | 1 220 $cm^{-1}$ |
| | 1 140 $cm^{-1}$ | 1 150 $cm^{-1}$ |

| | | |
|---|---|---|
| RMN ($D_2O$) | 3,15 ppm | (m, 4H) |
| | 6,3 ppm | (d, 1H) |
| | 6,45 ppm | (dded,1H) |
| | 7,5 ppm | (d, 1H) |

Analyse $C_6H_9NO$, HCl = 147,6
Calculé : C % 48,82 H % 6,82 N % 9,49
Trouvé : C % 48,70 H % 6,90 N % 9,40

Exemple 9 — Préparation du chlorhydrate de l'orthochlorophényl-2 éthylamine

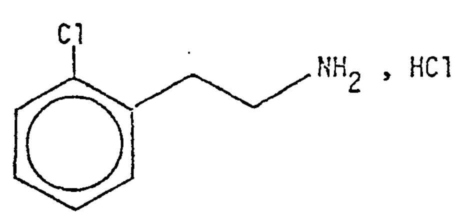

Stade a — N-orthochlorobenzylidène, aminométhylphosphonate de diéthyle

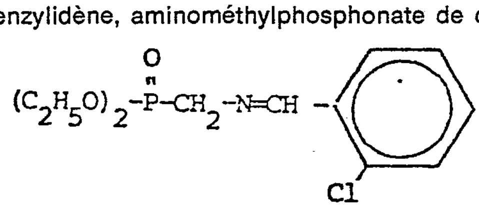

A 16,7 g (0,1 mole) d'aminométhylphosphonate de diéthyle en solution dans 200 ml de toluène, on ajoute, à température ambiante, goutte à goutte, 14 g (0,1 mole) de chloro-2 benzaldéhyde. A la fin de l'addition, on poursuit l'agitation pendant 30'. L'eau formée au cours de la réaction est éliminée par décantation. La phase toluénique est lavée avec 50 ml d'une solution aqueuse saturée de chlorure de sodium, séchée sur sulfate de sodium puis évaporée. On obtient 29 g (rendement 100 %) de N-orthochlorobenzylidène aminométhylphosphonate de diéthyle, sous forme d'une huile jaune monotache en CCM (plaque de silice,éluant AcOEt, rf = 0,45).

IR (film)
- C = N 1 635 $cm^{-1}$
- P = O 1 250 $cm^{-1}$
- P—O—C 1 060 $cm^{-1}$-1 030 $cm^{-1}$

RMN ($CDCl_3$) δ/TMS
- 1,35 ppm (t, 6H)
- 4,2 ppm (m, 6H)
- 7,1 à 7,5 ppm (m, 3H)
- 8 ppm (m, 1H)
- 8,7 ppm (d, 1H)

Stades b, c — Béta-(orthochlorophényl), N-vinyl, phosphoramidate d'éthyle

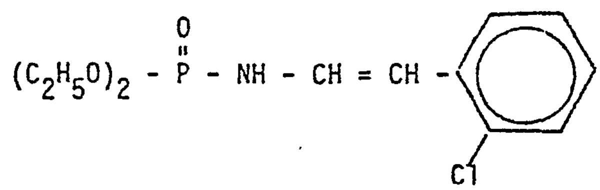

A une suspension de 4,8 g (0,1 mole) d'hydrude de sodium (à 50 % dans de l'huile) dans 10 ml de THF, on additionne goutte à goutte 28,95 g (0,1 mole) de l'imine préparée ci-dessus en solution dans 40 ml de THF. Après la fin de l'addition, durant laquelle la température s'élève de 20 à 30 °C, on porte le milieu à 45 °C pendant 2 heures. Après retour à température ambiante, on hydrolyse le milieu réactionnel en le versant dans 500 ml de solution aqueuse saturée de chlorure d'ammonium puis on extrait à l'éther isopropylique. Les phases éthérées réunies lavées avec une solution saturée de chlorure de sodium, séchées sur sulfate de sodium puis évaporées, conduisent à une huile jaune qui se concrétise par trituration avec de l'hexane. Après filtration du précipité, lavage de celui-ci avec un mélange éther isopropylique-hexane (10,90) et séchage sous vide, on obtient 21,7 g (75 % de rendement par rapport à l'aminométhylphosphonate de diéthyle) de béta (orthochlorophényl) N-vinyl, phosphoramidate de diéthyle sous forme de cristaux jaunes.

F = 98 °C

IR (pastille KBr)
- 3 400 $cm^{-1}$
- 3 150 $cm^{-1}$
- 1 650 $cm^{-1}$
- 1 430 $cm^{-1}$
- 1 240 $cm^{-1}$
- 1 020 $cm^{-1}$

RMN ($CDCl_3$) δ/TMS
- 1,3 ppm (t, 6H)
- 4,1 ppm (d,q,4H)
- 6 à 6,5 ppm (m,2H)
- 6,8 à 7,5 ppm (m, 5H)

Analyse : $C_{12}H_{17}ClNO_3P$ = 289,7
Calculé : C % 49,74 H % 5,91 N % 4,83
Trouvé : C % 49,54 H % 5,9 N % 4,80

Stade d — N-(orthochlorophényl-2 éthyl) phosphoramidate de diéthyle

A partir de 0,05 mole du phosphoramidate préparé précédemment et de 0,05 mole de borohydrure de sodium et en opérant comme à l'exemple 1, on obtient 14,6 g (rendement de la réduction 100 %) de N-(orthochlorophényl-2 éthyl) phosphoramidate de diéthyle sous forme d'une huile incolore.

IR (film)
- 3 300 $cm^{-1}$
- 1 250 $cm^{-1}$
- 1 050 $cm^{-1}$

RMN ($CDCl_3$) δ/TMS
- 1,3 ppm (t, 6H)
- 3 ppm (m, 5H) après échange avec $CD_3O$ (m, 4H)
- 4 ppm (qd, 4H)
- 7,2 ppm (m, 4H)

Stade e — Chlorhydrate de l'orthochlorophényl-2 éthylamine

Le phosphoramidate obtenu ci-dessus traité par l'acide chlorhydrique aqueux dans les conditions décrites à l'exemple 1, fournit, après basification et extraction à l'éther isopropylique, l'amine référencée qui est chlorhydratée par addition d'une solution éthanolique d'acide chlorhydrique gazeux.

Après filtration du précipité formé, lavage à l'éther isopropylique et séchage à 50 °C sous vide, on obtient 8,65 g (rendement 90 %) de chlorhydrate d'orthochlorophényl-2 éthylamine sous forme de cristaux blancs.

F = 145 °C

IR (pastille KBr)
- 3 300 $cm^{-1}$
- 2 900 à 3 000 $cm^{-1}$
- 1 575 $cm^{-1}$
- 1 475-1 450 $cm^{-1}$
- 1 230 $cm^{-1}$

RMN ($D_2O$)
- 3,25 ppm (m, 4H)
- 7,4 ppm (m, 4H)
- pic de l'eau à 4,5 ppm

Analyse $C_8H_{10}NCl$, HCl = 192,089
Calculé : C % 50,02 H % 5,77 N % 7,29
Trouvé : C % 49,85 H % 5,66 N % 7,21

## Exemple 10 — Préparation du chlorhydrate de (thiényl-2)-2 éthylamine

Stade a — N-thiénylidène-2, aminométhyl, phényl-phosphinate d'isopropyle

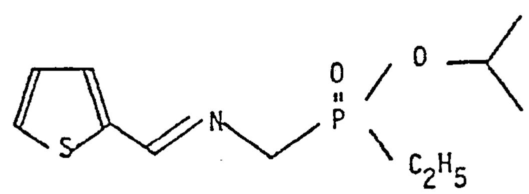

On prépare 0,1 mole du produit référencé en opérant comme décrit à l'exemple 1.

IR (film) C = N
- 1 625 $cm^{-1}$
- 1 430 $cm^{-1}$
- 1 200 $cm^{-1}$
- 980 $cm^{-1}$

RMN dans $CDCl_3$ δ/TMS
- 1,4 ppm (d de d, 6H)
- 4,15 ppm (d, 2H)
- 4,75 ppm (m, 1H)
- 7 à 8 ppm (m, 8H)
- 8,25 ppm (d, 1H)

Stades b, c — Phénylphosphonate d'isopropyle, N-[béta-(thiényl-2) vinyl] amide

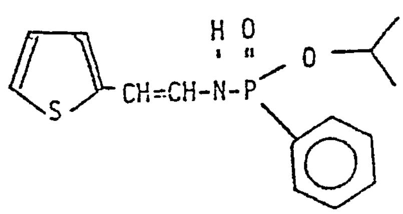

A partir de 0,1 mole d'imine préparée ci-dessus et en opérant comme à l'exemple 1, on obtient 18,6 g (rendement 60,5 %) du produit référencé sous forme de cristaux

F = 125 °C

| | |
|---|---|
| IR (pastille KBr) | 3 400-3 150 $cm^{-1}$ |
| | 1 650 $cm^{-1}$ |
| | 1 220 $cm^{-1}$ |
| | 1 000 $cm^{-1}$ |

| | | |
|---|---|---|
| RMN ($CDCl_3$) δ/TMS | 1,35 ppm | (d, 6H) |
| | 4,8 ppm | (m, 1H) |
| | 5,9 ppm | (m, 1H) |
| | 6,2 à 7 ppm | (m, 4H) |
| | 7 à 8 ppm | (m, 6H) dont 1 échangeable avec $D_2O$ |

Stade d — Phénylphosphonate d'isopropyle,N-[(thiényl-2)-2 éthyle] amide

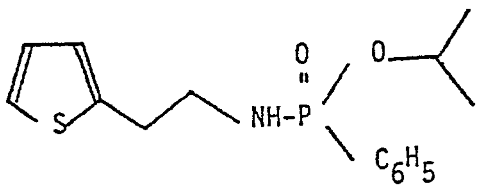

Le composé obtenu au stade précédent est réduit par le borohydrure de sodium dans l'alcool, en procédant comme à l'exemple 1. On obtient ainsi 18,6 g (rendement 60,2 % par rapport à l'aminométhyl phénylphosphinate) de phosphonate référencé sous forme d'une huile jaune clair.

| | |
|---|---|
| IR (film) | 3 400 $cm^{-1}$ |
| | 3 220 $cm^{-1}$ |
| | 2 980 $cm^{-1}$ |
| | 2 930 $cm^{-1}$ |
| | 2 870 $cm^{-1}$ |
| | 1 600 $cm^{-1}$ |
| | 1 440 $cm^{-1}$ |
| | 1 220 $cm^{-1}$ |
| | 990 $cm^{-1}$ |

| | | |
|---|---|---|
| RMN ($CDCl_3$) δ/TMS | 1,3 ppm | (d de d, 6H) |
| | 3 ppm | (m, 5H) dont 1 échangeable avec $D_2O$ |
| | 4,65 ppm | (m, 1H) |
| | 6,7 à 7,9 ppm | (m, 8H) |

Stade e — Chlorhydrate de (thiényl-2)-2 éthylamine

Le phosphonate obtenu au stade d (18,6 g) est traité par une solution d'acide chlorhydrique gazeux dans l'éther isopropylique, une nuit à température ambiante pour fournir 9 g (rendement 55 % par rapport à l'aminométhylphénylphosphinate) de chlorhydrate de (thiényl-2)-2 éthylamine dont les caractéristiques physiques, spectrales et analytiques sont identiques à celles du produit obtenu à l'exemple 1.

**Revendications**

1. Procédé de préparation d'éthylamines β-cyclo-substituées de formule

$$Ar—CH_2—CH_2—NH_2 \qquad (I)$$

dans laquelle Ar représente un radical aromatique hétérocyclique ou non éventuellement mono- ou polysubstitué par des groupes halogéno, nitro, amino, cyano, carboxy, alcoyle, halogéno-alcoyle, alcoxy, halogéno-alcoxy ou phényle, cractérisé en ce que l'on condense un dérivé de formule

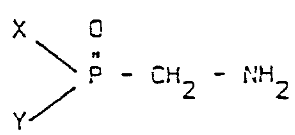

(II)

dans laquelle X et Y sont identiques ou différents et un radical alcoyle, aryle, alcoxy, aryloxy, dialkylamino ou diarylamino, avec un composé carbonylé de formule

$$Ar—CHO \qquad (III)$$

dans lequel Ar est tel que défini précédemment pour obtenir un composé de formule

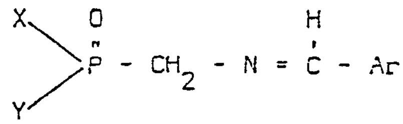

(IV)

dans laquelle X, Y et Ar sont tels que définis précédemment, qui est traité par une base de formule $B^{\ominus} M^{\oplus}$ pour conduire à un carbanion de formule

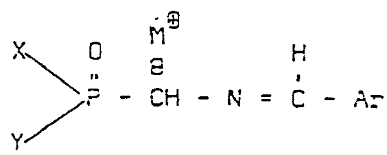

(V)

qui se transforme en dérivé de formule sous l'action de la chaleur

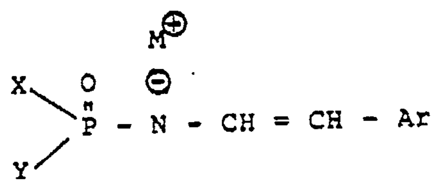

(VI)

pour aboutir, après reprise par l'eau, en un composé de formule

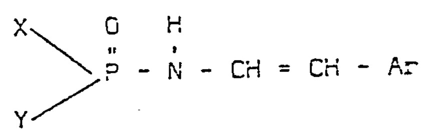

(VII)

que l'on traite par un agent réducteur pour obtenir le composé de formule

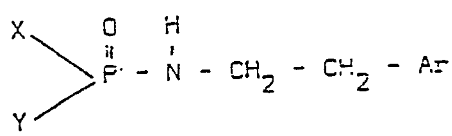

(VIII)

qui est soumis à l'action d'un acide pour conduire au dérivé de formule I.

2. Procédé selon la revendication 1, caractérisé en ce que Ar est un groupe thiényle, furfuryle, pyridyle, phényle ou naphtyle.

3. Procédé selon la revendication 1, caractérisé en ce que $M^{\oplus}$ est un métal alcalin ou alcalino-terreux et $B^{\ominus}$ est un carbanion choisi parmi les groupes chargés négativement correspondants, hydrogène, hydroxy, aminno, mono- ou dialkylamino, alkyle linéaire ou ramifié, alkoxy linéaire ou ramifié où la base $B^{\ominus} M^{\oplus}$ est un composé organomagnésien ou un composé organosodé.

4. Procédé selon la revendication 3, caratérisé en ce que $M^{\oplus}$ est un métal choisi parmi le sodium, le lithium, le potassium et le magnésium.

5. Procédé suivant la revendication 1, caractérisé en ce que la transformation du carbanion en dérivé VI s'effectue à une température comprise entre − 78 °C et + 150 °C.

6. Procédé suivant la revendication 5, caractérisé en ce que la réaction s'effectue à une température choisie en fonction de la base dans une plage plutôt haut de gamme.

7. Procédé suivant les revendications 5 et 6, caractérisé en ce que la réaction s'effectue dans un solvant organique tel que des éthers linéaires ou cyliques, des hydrocarbures aromatiques, des alcools, des amides ou des sulfoxydes.

8. Procédé selon la revendication 7, caractérisé en ce que le solvant est choisi parmi le tétrahydrofuranne, le benzène, le toluène, les xylènes, l'éthanol, le diméthylformamide et le diémthylsulfoxyde.

9. Procédé suivant la revendication 1, caractérisé en ce que la réduction du dérivé de formule VII est effectuée par un borohydrure de métal alcalin notamment le borohydrure de sodium ou le borohydrure de potassium.

**Claims**

1. Process for the preparation of β-cyclo-substituted ethylamines having the formula

$$Ar—CH_2—CH_2—NH_2 \qquad (I)$$

in which Ar represents a heterocyclic or non heterocyclic aromatic radical optionally mono- or polysubstituted with halo, nitro, amino, cyano, carboxy, alkyl, halo-alkyl, alkoxy, haloalkoxy or phenyl group comprising : condensing a derivative of the formula

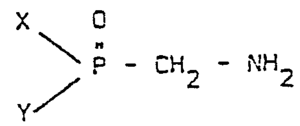

(II)

in which X an Y are identical or different and represent an alkyl, aryl, alkoxy, aryloxy,dialkylamino or diarylamino radical, with a carbonyl compound of the formula

$$Ar—CHO \qquad (III)$$

in which Ar is such as defined above, to give a compound of the formula

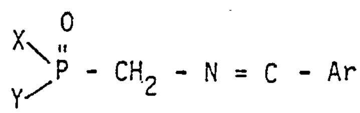

(IV)

in which X, Y and Ar have the above defined meanings ; treating compound (IV) with a base of the formula $B^{\ominus}$ $M^{\oplus}$ to give a carbanion of the formula

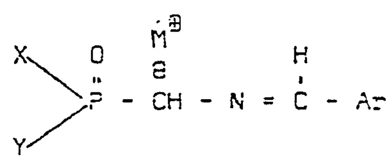

(V)

converting compound (V) to a derivative of the formula

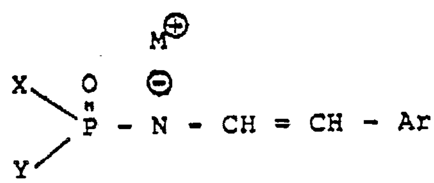

(VI)

under the action of heat, to give, after taking up into water, a compound of the formula

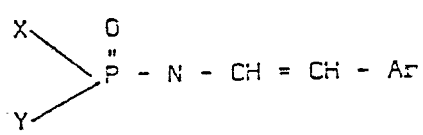

(VII)

and then treating compound (VII) with a reducing agent, to give the compound of the formula

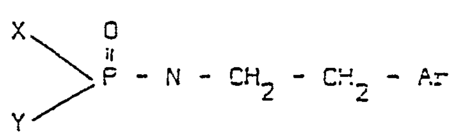

(VIII)

and submitting compound (VIII) to the action of an acid, to give the derivative of the formula (I).

2. Process as claimed in claim 1, wherein Ar is a thienyl, furyl, pyridyl, phenyl or naphthyl group.

3. Process as claimed in claim 1, wherein $M^{\oplus}$ is an alcali or alcaline earth metal and $B^{\ominus}$ is a carbanion selected from the negatively charged group corresponding to hydrogen, hydroxy, amino, mono- or dialkylamino, straight- or branched chain alkyl or straight-or branched chain alkoxy or the base $B^{\ominus}$ $M^{\oplus}$ is an organomagnesium compound or an organosodium compound.

4. Process as claimed in claim 3, wherein $M^{\oplus}$ is a metal selected from sodium, lithium, potassium and magnesium.

5. Process as claimed in claim 1, wherein the conversion of the carbanion to the derivative (VI) is effected at a temperature comprised between − 78 °C and + 150 °C.

6. Process as claimed in claim 5, wherein the reaction is effected at a temperature selected as a function of the base within the higher temperatures of said range.

7. Process as claimed in claim 5 and 6, wherein the reaction is effected within an organic solvent selected from straightchain and cyclic ethers, aromatic hydrocarbons, alcohols, amides and sulfoxides.

8. Process as claimed in claim 7, wherein the solvent is selected from tetrahydrofuran, benzene, toluene, xylenes, ethanol, dimethylformamide and dimethylsulfoxide.

9. Process as claimed in claim 1, comprising effecting the reduction of the derivative of the formula (VII) with an alkali metal borohydride, tipycally selected from sodium borohydride and potassium borohydride.

**Patentansprüche**

1. Verfahren zur Herstellung von β-cyclo-substituierten Ethylaminen der Formel

$$Ar—CH_2—CH_2—NH_2 \qquad (I)$$

in der Ar einen aromatischen Rest darstellt, der heterocyclisch oder nicht sein kann und gegebenenfalls einfach oder mehrfach durch eine Halogen-, Nitro-, Amino-, Cyano-, Carboxy-, Alkyl-, Halogenalkyl-, Alkoxy-, Halogenalkoxy- oder Phenyl-Gruppe substituiert sein kann, dadurch gekenzeichnet, daß man ein Derivat der Formel

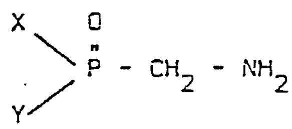

(II)

in der X und Y gleich oder verschieden sind und einen Alkyl-, Aryl-, Alkoxy-, Aryloxy-, Dialkylamino- oder Diarylamino-Rest darstellen, mit einer Carbonylverbindung der Formel

$$Ar—CHO \qquad (III)$$

kondensiert, in der Ar wie vorher definiert ist, um eine Verbindung der Formel

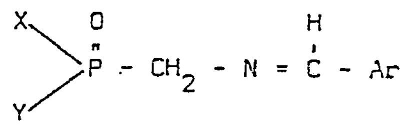

(IV)

zu erhalten, in der X, Y und Ar wie oben definiert sind, die mit einer Base der Formel $B^{\ominus} M^{\oplus}$ behandelt wird und zu einem Carbanion der Formel

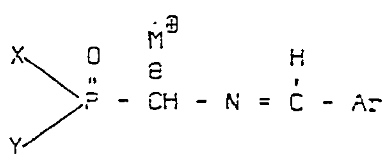

(V)

führt, das sich unter Einwirkung von Wärme in ein Derivat der Formel

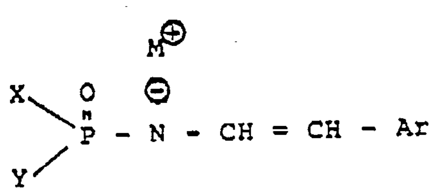

(VI)

umlagert und nach Aufnahme von Wasser zu einer Verbindung der Formel

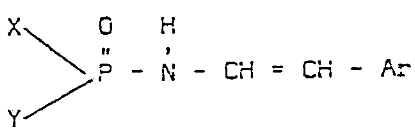

(VII)

führt, die man mit einem Reduktionsmittel behandelt, um die Verbindung der Formel

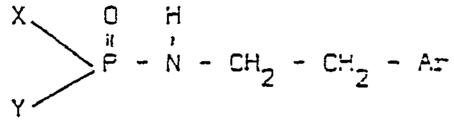

(VIII)

zu erhalten, die einer Säurebehandlung unterworfen wird, um zu einem Derivat der Formel I zu führen.

2. Verfahren gemäß Anspruch 1, dadurch gekennzeichnet, daß Ar eine Thienyl-, Furyle-, Pyridyl-, Phenyl- oder Naphthyl-Gruppe ist.

3. Verfahren gemäß Anspruch 1, dadurch gekennzeichnet, daß $M^{\oplus}$ ein Alkali- oder Erdalkalimetall ist und $B^{\ominus}$ ein Carbanion aus den entsprechenden negativ geladenen Wasserstoff-, Hydroxy-, Amino-, Mono- oder Dialkylamino-, linearen oder verzweigten Alkyl-, linearen oder verzweigten Alkoxy-Gruppen ist oder die Base $B^{\ominus}$ $M^{\oplus}$ eine magnesiumorganische oder eine natriumorganische Verbindung ist.

4. Verfahren gemäß Anspruch 3, dadurch gekennzeichnet, daß $M^{\oplus}$ ein Metall aus der Gruppe Natrium, Lithium, Kalium und Magnesium ist.

5. Verfahren gemäß Anspruch 1, dadurch gekennzeichnet, daß die Umlagerung des Carbanions in das Derivat VI bei einer Temperatur zwischen − 78 °C und + 150 °C erfolgt.

6. Verfahren gemäß Anspruch 5, dadurch gekenzeichnet, daß die Reaktion bei einer Temperatur erfolgt, die in Abhängigkeit von der Base eher im oberen Teil des Bereiches liegt.

7. Verfahren gemäß Ansprüchen 5 und 6, dadurch gekennzeichnet, daß die Reaktion in einen organischen Lösungsmittel wie linearen oder cyclischen Ethern, aromatischen Kohlenwasserstoffen, Alkoholen, Amiden oder Sulfoxiden erfolgt.

8. Verfahren gemäß Anspruch 7, dadurch gekennzeichnet, daß das Lösungsmittel aus der Gruppe Tetrahydrofuran, Benzol, Toluol, Xylol, Ethanol, Dimethylformamid und Dimethylsulfoxid ausgewählt wird.

9. Verfahren gemäß Anspruch 1, dadurch gekennzeichnet, daß die Reduktion des Derivats der Formel VII unter Einwirkung von Alkalimetallborhydriden, insbesondere Natrium- oder Kaliumborhydriden erfolgt.